# EUROPEAN PATENT APPLICATION

(11) **EP 2 932 975 A1**
(43) Date of publication of application: **21.10.2015**
(21) Application number: 15156095.0
(22) Date of filing: 14.04.2010
(51) Int. Cl.: A61K 38/13, A61P 27/02

(54) **Cyclosporin composition for use in treating blurred vision**

(30) Priority: 14.04.2009 US 169196 P
(62) Divisional of application: 10714144.2
(71) Applicant: ALLERGAN, INC., Irvine, CA 92612 (US)
(72) Inventor: Schiffman, Rhett M., Laguna Beach, CA California 92651 (US)
(74) Representative: Hoffmann Eitle

(57) **Abstract**

The present invention provides a cyclosporin composition for use in a method of treating blurred vision by topical administration to the eye.

## Description

### FIELD OF THE INVENTION

The present invention relates to a cyclosporin composition for use in treating blurred vision.

### DETAILED DESCRIPTION OF THE INVENTION

### Cyclosporin

Cyclosporins are a group of nonpolar cyclic oligopeptides with known immunosuppressant activity. Cyclosporin A, along with several other minor metabolites, as well as cyclosporin B, C, D, E, F, G, H ,I, J, K, L, M, N, O, P, Q, R, S, T, U, V, W, X, Y and Z, have been identified. In addition, derivatives, salts and the like of such cyclosporins and a number of synthetic analogs have been prepared and may be useful in the present invention. The use of cyclosporin A and cyclosporin A derivatives to treat ophthalmic conditions has been the subject of various patents, for example Ding et al U.S. Patent 5,474,979; Garst U.S. Patent 6,254,860; and Garst U.S. 6,350,442, this disclosure of each of which is incorporated in its entirely herein by reference

In general, commercially available cyclosporins may contain a mixture of several individual cyclosporins which all share a cyclic peptide structure consisting of eleven amino acid residues with a total molecular weight of about 1,200, but with different substituents or configurations of some of the amino acids.

As used here, a "cyclosporin composition" includes any individual member of the cyclosporin group, salts thereof, derivatives thereof, analogs thereof and mixtures thereof, as well as mixtures of two or more individual cyclosporins salts thereof, derivatives thereof, analogs thereof and mixtures thereof.

In one embodiment, the cyclosporin composition comprises cyclosporin A, a derivative of cyclosporin A, a salts of cyclosporin A, and/or mixtures thereof.

The chemical structure for cyclosporin A is represented by Formula 1

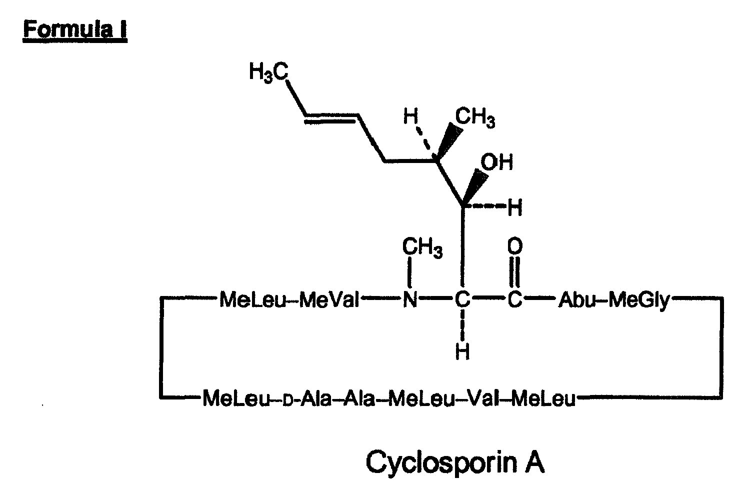

As used here the term "derivatives" of a cyclosporin refer to compounds having structures sufficiently similar to the cyclosporin so as to function in a manner substantially similar to or substantially identical to the cydosporin, for example, cyclosporin A, in the present methods. Included, without limitation, within the useful cyclosporin A derivatives are those selected from ((R)-methylthio-Sar)³-(4'-hydroxy-MeLeu) cyclosporin A, ((R)-(Cyclo)alkylthio-Sar)³-(4'-hydroxy-MeLeu)⁴-cyclosporin A, and ((R)-(Cyclo)alkylthio-Sar)³-cyclosporin A derivatives described below.

These cyclosporin derivatives are represented by the following general formulas (II), (III), and (IV) respectively: 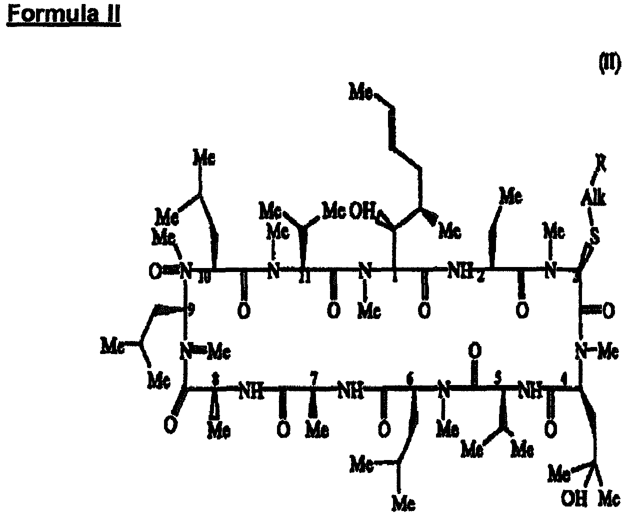 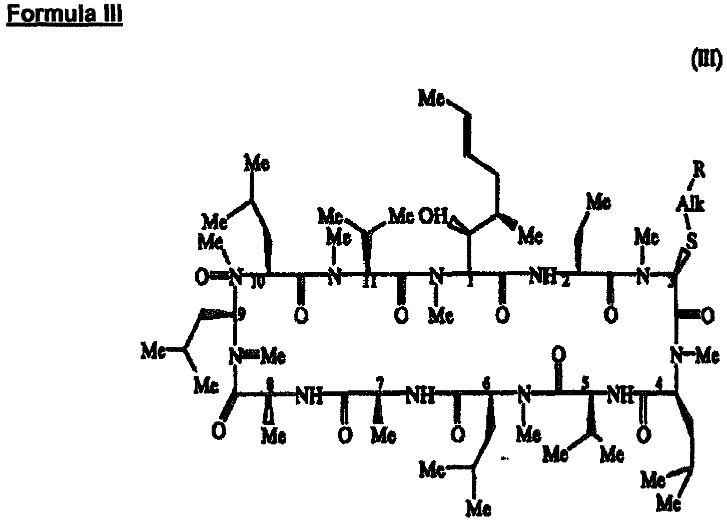 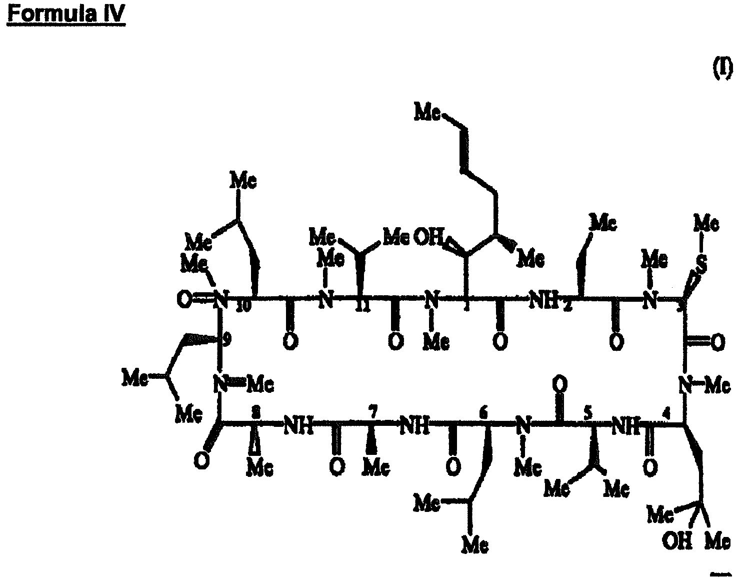 wherein Me is methyl; Alk is 2-6C alkylene or 3-6C cycloalkylene; R is OH, COOH, alkoxycarbonyl, -NR₁R₂ or N(R₃)-(CH₂)-NR₁R₂; wherein R₁,R₂ is H, alkyl, 3-6C cycloalkyl, phenyl (optionally substituted by halo, alkoxy, alkoxycarbonyl, amino, alkylamino or dialkylamino), benzyl or saturated or unsaturated heterocyclyl having 5 or 6 members and 1-3 heteroatoms; or NR₁R₂ is a 5 or 6 membered heterocycle which may contain a further N, O or S heteroatom and may be alkylated; R₃ is H or alkyl and n is 2-4; and the alkyl moieties contain 1-4C.

The cyclosporin composition contains from about 0.005% to about 15% or about 20% or more cyclosporin by weight of the composition. In one embodiment, the composition contains cyclosporin in an amount of about 0.02% to about 1% or about 5% or about 10% by weight of the composition. The choice of a particular amount of cyclosporin is to be made in accordance with factors well known in the medicinal arts, including mode of administration, the size and condition of the human or animal and the type and severity of the condition to be treated.

The cyclosporin compositions may be liquids, suspensions, emulsions, semi-solids, capsules, gels, lotions, creams and the like.

For example, cyclosporin can be combined with carriers which form emulsions upon mixing with water. Such emulsions are described, for example, and without limitation, in Cavanak U.S. Pat. No. 4,388,307, the disclosure of which is hereby incorporated in its entirety herein by reference. Carriers, for example, and without limitation, glyceride carriers, may assist in alleviating problems of physical instability such as precipitation of the cyclosporin from solution, and may also enable higher blood plasma concentrations, if desired.

In one embodiment, the cyclosporin compositions include hydrophobic components. Any suitable hydrophobic component may be employed in the present invention. In one embodiment, the cyclosporin may be solubilized in the hydrophobic component. In another embodiment, the hydrophobic component may be considered as comprising a discontinuous phase in the cyclosporin compositions, for example, oil-in-water emulsions.

The hydrophobic component may be present in an effective amount, for example, in an amount of about 0.1 % or about 1.0% to about 1.5% or about 10% by weight or more of the composition.

In one embodiment, the hydrophobic component comprises one or more oily materials. Examples of useful oil materials include, without limitation, vegetable oils, animal oils, mineral oils, synthetic oils and the like and mixtures thereof. In one embodiment, the hydrophobic component comprises one or more higher fatty acid glycerides. One can obtain excellent results with castor oil.

In another embodiment, cyclosporin compositions may comprise cyclosporin in admixture with an emulsifying amount of a fatty acid glyceride, such as castor oil and the like, and a surfactant, such as polysorbate 80. Such compositions are described in Ding et al U.S. Patent 5,474,979, the disclosure which is hereby incorporated in its entirety herein by reference. Also see Kaswan U.S. Patent 4,649,047 and Kaswan U.S. Patent 5,411,952, the disclosure of each of which is hereby incorporated in its entirety herein by reference.

In one embodiment, the presently useful compositions are self-emulsifying which, when exposed to an aqueous medium, form fine oil-in-water emulsions with little or no agitation. The property of self-emulsification permits such formulations to be administered in concentrated form, as for example in a hard gelatin or soft elastic capsules, with the expectation that a fine emulsion will be formed in the digestive tract. Additionally, emulsions may be prepared by combining a self-emulsifying pre-concentrate with an aqueous medium.

Previously disclosed self-emulsifying systems include those in which a cyclosporin is combined with mixtures of (i) medium-chain triglycerides and nonionic surfactants, (ii) vegetable oils and partial glycerides, such as polyglycolized glycerides or medium-chain mono- and diglycerides, or (iii) vegetable oils and nonionic surfactants such as polysorbate 80 or PEG-25 glyceryl trioleate.

In certain self-emulsifying formulations, a "microemulsion preconcentrate" of a cyclosporin is formed by combining the cyclosporin with (I) a hydrophilic phase, (II) a lipophilic phase, and (III) a surfactant, as well as optional thickeners, antioxidants or other excipients. Examples of such compositions are disclosed in Hauer et al U.S. Patent 5,342,625, the disclosure which is hereby incorporated in its entirety herein by reference.

In addition, suitable compositions may include cyclosporins in combination with a hydrophilic solvent phase and one or more surfactants, but not containing lipophilic solvents. Such cyclosporin-containing formulations may be stable, simple to prepare, and have good pharmacokinetic properties.

As used herein, the terms "binary system", "binary composition" and "binary system of excipients" denote those cyclosporin compositions which contain, in addition to cyclosporin, a combination of at least one hydrophilic solvent and at least one surfactant, but which lack a lipophilic solvent. Such compositions may be supplemented with additional adjuvants and still be considered "binary", so long as they do not include a lipophilic solvent phase.

To prepare such pharmaceutical compositions, a binary system is combined with a cyclosporin. The hydrophilic phase may comprise one or more of the known pharmaceutically acceptable hydrophilic solvents or excipients that are capable of solubilizing the cyclosporin. Suitable classes of hydrophilic compounds include, for example and without limitation, pharmaceutically acceptable alcohols including the polyethylene glycols.

Examples of hydrophilic phase components useful in the presently useful compositions include, but are not limited to, water, ethanol, benzyl alcohol, propylene glycol, low molecular weight polyethylene glycols having a molecular weight of up to about 1,000, glycol, dimethyl isosorbide and the like and mixtures thereof.

The compositions may be prepared as semi-solids and placed into hard gelatin rather than soft elastic capsules, to allow for the use of ethanol and similar solvents.

The hydrophilic phase, comprising one or more hydrophilic solvents, typically comprises about 10% to about 90% by weight of the pharmaceutical composition. The precise amount used will vary depending on the nature of the hydrophilic compound or compounds used, the amount of cyclosporin component present, the dosage form, the condition being treated and other factors known in the art. Preferably the hydrophilic phase comprises about 20% to about 80%, and more preferably about 30% to about 60%, by weight of the composition. Where non-aqueous hydrophilic components are used, water can be included in the formulation at levels varying from about 0.5% to about 10%, or preferably from about 1% to about 5%, based on total weight of the composition.

Any of the known pharmaceutically acceptable surfactants may be used, including nonionic, anionic, cationic, and combinations thereof. Nonionic surfactants are preferred, and especially those surfactants having a hydrophile/lipophile balance (HLB) of 10 or more. Alternatively, certain combinations of high- and low-HLB surfactants may be utilized; preferably, such mixed surfactants are used in ratio such that the aggregate surfactant HLB (when weighted according to proportions used) remains in excess of 10.

Examples of suitable surfactants include, but are not limited to, polyoxyethylene derivatives of natural or hydrogenated vegetable oils such as castor oil; polyoxyethylene-sorbitan fatty acid esters, such as mono-, di- and tri-lauryl, palmityl, stearyl and oleyl esters; alkyl/dialykyl sulfate, sulfonate or sulfosuccinate salts such as sodium lauryl sulfate and dioctyl sodium sulfosuccinate; polyoxyethylene fatty acid esters; phospholipids such as lecithins; transesterification products of natural vegetable oil triglycerides and polyalkylene polyols; sorbitan fatty acid esters; pentaerythritol fatty acid esters; polyoxyethylene glycol alkyl ethers and esters; and the like. The surfactants may be used alone or in combination.

Examples of specific surfactants which may be used include, without limitation, polyoxyethylene castor oil derivatives, such as polyoxyethylene glycerol triricinoleate polyoxyl 35 castor oil (CREMOPHOR® EL, available from BASF Corporation), and polyoxyl 40 hydrogenated castor oil (CREMOPHOR® RH40, available from BASF Corporation); mono-fatty acid esters of poloxyethylene (20) sorbitan, such as polyoxyethylene (20) sorbitan monooleate (TWEEN® 80), polyoxyethylene (20) sorbitan monostearate (TWEEN® 60), polyoxyethylene (20) sorbitan monopalmitate (TWEEN® 40), and polyoxyethylene (20) sorbitan monolaurate (TWEEN® 20) (all available from ICI Surfactants, Wilmington, Del.); polyoxyethylene glycol 200 monostearate (MYRJ® 52, available from Calgene Chemicals, Skokie, III.); polyglycerol esters with a HLB of 10 or greater, such as decablyceryl mono- and dioleate and the like; and mixtures thereof.

In some instances (as when the compositions are prepared as semi-solids), it may be advantageous to use at least one additional low-HLB surfactant along with one or more of the above high-HLB surfactant. Examples of low-HLB auxiliary surfactants which may be used include, but are not limited to, polyglycerol oleates (such as CAPROL® 10G40); lecithins; glyceryl monooleate or monolinoleate mixtures (such as MYVEROL® 18-99 or 18-92); propylene glycol laurate; and sorbitan oleates such as sorbitan monooleate (SPAN® 80), sorbitan trioleate (SPAN® 85), and sorbitan sesquioleate (SPAN® 20) (all available from ICI Surfactants, Wilmington, Del.). The surfactant phase may comprise about 10% to 90% by weight of the composition. Preferably the surfactant comprises about 20% to about 70% of the composition, and more preferably about 40% to about 60%, by weight.

If desired, the cyclosporin composition may additionally comprise other pharmaceutically acceptable excipients, such as tonicity components, buffer components, polyelectrolyte components, thickeners, fillers, diluents, flavoring agents, coloring agents, antioxidants, preservatives, such as antibacterial or antifungal agents, acids and/or basis to adjust pH, and the like and mixtures thereof. Such additives, if present, may typically comprise about 0.01% to about 10% by weight of the composition. Such additives include those additives which are conventional and/or well known for use in similar pharmaceutical compositions. For example, suitable thickening agents include any of those known in the art, as for example pharmaceutically acceptable polymers and/or inorganic thickeners. Such agents include, but are not limited to, polyacrylate homo- and co- polymers; celluloses and cellulose derivatives; polyvinyl pyrrolidones; polyvinyl resins; silicates; and the like and mixtures thereof.

Additional formulations of cyclosporin are described in and well known in the art.

When desired, the cyclosporin compositions may be prepared as semi-solid rather than liquid formulations by addition a greater proportion of appropriate thickening or solidifying agents. As noted above, such preparations may be particularly useful as fills for hard gelatin (as opposed to soft gelatin) capsules. Solidifiers suitable for the preparation of semi-solid compositions Include, but are not limited to, polyethylene glycols having a molecular weight of more than about 1,000, such as PEG 1450 and PEG 3350; stearyl alcohol; and colloidal silicon dioxide (CAB-O-SIL® M-5, available from Cabot, Tuscola, III.). A semi-solid state may be often obtained by adding between about 8% or about 10% and about 15% or about 25% by weight solidifying agent. The actual amount of solidifying agent needed will depend on the physical characteristics of the other excipients which are present.

The cyclosporin compositions of the invention may be formulated for topical or systemic administration. For systemic administration, for example, oral administration of a suspension formed by mixing a cyclosporin with an aqueous medium such as water, milk or juice; a cyclosporin composition placed in a soft elastic or hard gelatin capsule; parenteral administration including intravenous, intramuscular, intraperitoneal, intrastemal, subcutaneous and intraarticular injection or infusion of a cyclosporin composition; and/or topical administration methods, such as topical administration of ointments, drops, solutions, suspensions or emulsions including a cyclosporin.

Topical formulations may be prepared directly, or by combining a cyclosporin concentrate with a diluent, for example, an aqueous diluent. Such topical formulations may include additional excipients as necessary, for example, to modify consistency of the rate of absorption of the cyclosporin.

### Methods of treating

The cyclosporin compositions of the invention may be administered to treat a patient having any of the conditions described herein. To "treat," as used here, means to deal with medically. It includes administering the compositions of the invention to prevent a condition, to cause it to improve, and to lessen its severity.

In one embodiment, the method of the invention may be used to treat blurred vision. "Blurred vision" refers to any distortion of vision characterized by a lack of sharpness of retinal focus. Refractive error, such as that associated with distortions in the shape of the lens, is often the cause, but other diseases and conditions of the eye may blur vision, as well; hence, the method of the invention may be used to treat a patient with blurred vision whatever the disease or condition of the eye that causes it. The blurred vision may be naturally occurring, the result of a disease process in the eye, or may be iatrogenic, the result of surgical or other therapeutic intervention. Laser in situ keratomileusis (LASIK) or photorefractive keratectomy (PRK) are two common causes of iatrogenic blurred vision: although the goal of such procedures is to improve visual acuity, a patient may still experience some blurred vision, particularly in the period of recovery after the procedure; one can treat such patients with the cyclosporin compositions disclosed here in order to shorten that period and to improve vision.

In another embodiment, the method of the invention may be used to treat sensitivity to light. "Sensitivity to light" refers to any condition in which a patient experiences discomfort on exposing his eyes to light, particularly to bright light, wherein the discomfort is the result of a disease or condition of the eye. The discomfort may be painful, and may be accompanied by excessive tearing and behavior seeking to avoid the source of the light. In patients experiencing sensitivity to light the photoreceptors in the retina are often over stimulated, the result of excessive light entering the eye; this might occur in patients with damage to the retina, cornea, or the constriction mechanisms of the pupil.

In another embodiment, the method of the invention may be used to treat keratocyte loss. Corneal surgery, including LASIK and PRK, may result in a decrease in keratocyte density, and this decrease may persist for years following the surgery. Keratocytes are involved in maintaining homeostasis of the eye and in repairing wounds to it; a persistent decrease in their number may compromise eye health. The method of the invention may be used to treat keratocyte loss, thereby maintaining good eye health

In another embodiment, the method of the invention may be used to increase goblet cell density on the eye. Corneal surgery, including LASIK and PRK, may result in a decrease in goblet cell density. Goblet cells are involved in lubricating the eye; a decrease in their number may compromise eye health. The method of the invention may be used to treat goblet cell loss, thereby maintaining good eye health.

In another embodiment, the method of the invention may be used to treat psychological distress following surgery on the eye. The inventors have discovered that patients administered cyclosporin after such surgery report a smaller amount of such distress.

The cyclosporin compositions may be administered topically and/or systemically. In a typical topical administration, 25 to 50 µl of the composition is administered to the eye.

The cyclosporin compositions may be administered once, twice, three times, four times, or more, daily. Where the cyclosporin composition is administered to a patient that will experience corneal surgery, the composition may be administered before it, as well.

### EXAMPLES

The method of the invention is further illustrated by the following examples.

### EXAMPLE I

Corneal surgeons performed LASIK on 137 patients, using an IntraLase® FS laser (Abbott Medical Optics, Santa Ana, California) to create a 150µm flap with superior hinge in both eyes. Eyes were divided into treatment and control groups, and dosed with either Restasis® (treatment) or Endura® (control) twice daily in a double-masked, randomized manner. Restasis® contains cyclosporin A 0.05% as the single active ingredient; it is approved in the United States for the increasing of tear production in patients whose tear production is presumed to be suppressed due to ocular inflammation associated with keratoconjunctivitis sicca. Endura® is an eye drop available over the counter for the treatment of dry eyes, consisting of a emulsion that is substantially similar to the vehicle used in Restasis®. In administering each dose, approximately 50 µl was instilled in the eye, and a smaller amount absorbed.

Patients were followed for 6 months and evaluated 28 days and 1 day prior to the surgery, and at 1 day, 2 days, 1 week, and one month after the surgery and at monthly intervals thereafter. Patients were administered a survey that asked whether they had "blurred vision," "poor vision" and "eyes that are sensitive to light," among other questions. Patients responded by indicating how frequently they experienced those symptoms, which were scored as follows:

**Table 1 - Symptom scores**

| Symptom | Score |
|---|---|
| All of the time | 4 |
| Most of the time | 3 |
| Half of the time | 2 |
| Some of the time | 1 |
| None of the time | 0 |

Results are shown below. In each table, the lower value, the less severe the symptom.

**Table 2 - Blurred vision scores**

| | Restasis® | Endura® | |
|---|---|---|---|
| | (N= 68) | (N= 69) | p-value [a] |
| Baseline | 0.2 | 0.1 | 0.145 |
| Month 1 | 0.5 | 0.7 | 0.262 |
| Month 3 | 0.2 | 0.5 | 0.041 |
| Month 4 | 0.1 | 0.4 | 0.068 |
| Month 6 | 0.2 | 0.4 | 0.138 |

**Table 3 - Poor vision scores**

| | Restasis® | Endura® | |
|---|---|---|---|
| | (N= 68) | (N= 69) | p-value [a] |
| Baseline | 0.4 | 0.2 | 0.208 |
| Month 1 | -0.1 | 0.3 | 0.049 |
| Month 3 | -0.1 | 0.3 | 0.014 |
| Month 4 | -0.2 | 0.2 | 0.032 |
| Month 6 | -0.1 | 0.3 | 0.031 |

**Table 4 - Sensitivity to light scores**

| | Restasis® | Endura® | |
|---|---|---|---|
| | (N= 68) | (N= 69) | p-value [a] |
| Baseline | 0.4 | 0.3 | 0.361 |
| Month 1 | 0.3 | 0.8 | 0.005 |
| Month 3 | 0.3 | 0.4 | 0.795 |
| Month 4 | 0.1 | 0.1 | 0.913 |
| Month 6 | 0 | 0.1 | 0.386 |

Patients were also evaluated using the National Eye Institute Refractive Error Questionnaire. Results were as follows. In each table, the higher the value, the less severe the symptom.

**Table 5 - Diurnal fluctuation**

| | Restasis® | Endura® | |
|---|---|---|---|
| | (N= 68) | (N= 69) | p-value [a] |
| Baseline | 80.3 | 82.6 | 0.551 |
| Month 1 | -1.1 | -5.6 | 0.357 |
| Month 3 | 5.6 | -0.1 | 0.226 |
| Month 4 | 8.8 | 1.2 | 0.074 |
| Month 6 | 6.4 | 2.6 | 0.397 |

**Table 6 - Distorted vision**

| | Restasis® | Endura® | |
|---|---|---|---|
| | (N= 68) | (N= 69) | p-value [a] |
| Baseline | 89 | 89.5 | 0.905 |
| Month 1 | -6.8 | -18.3 | 0.082 |
| Month 3 | -3.3 | -6.9 | 0.55 |
| Month 4 | 4.4 | -10.1 | 0.009 |
| Month 6 | 0.7 | -2.2 | 0.596 |

**Table 7 - Worry**

| | Restasis® | Endura® | |
|---|---|---|---|
| | (N= 68) | (N= 69) | p-value [a] |
| Baseline | 50.9 | 52.5 | 0.726 |
| Month 1 | 17 | 15 | 0.71 |
| Month 3 | 23.9 | 22.1 | 0.733 |
| Month 4 | 31.3 | 21.2 | 0.063 |
| Month 6 | 28.9 | 23.2 | 0.313 |

**Table 8 - Suboptimal correction**

| | Restasis® | Endura® | |
|---|---|---|---|
| | (N= 68) | (N= 69) | p-value [a] |
| Baseline | 91.5 | 82.1 | |
| Month 1 | 4.7 | 14.4 | 0.655 |
| Month 3 | 5.9 | 16.1 | 0.773 |
| Month 4 | 7.7 | 14.3 | 0.142 |
| Month 6 | 8.3 | 14.3 | 0.023 |

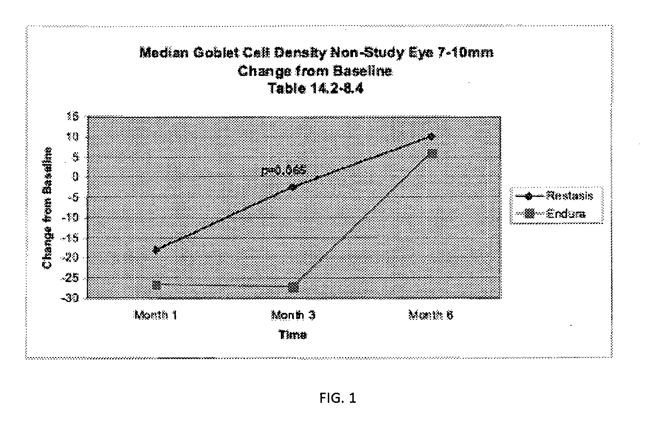

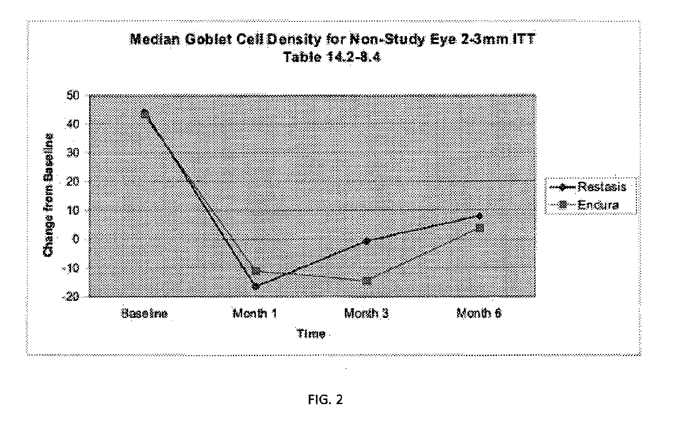

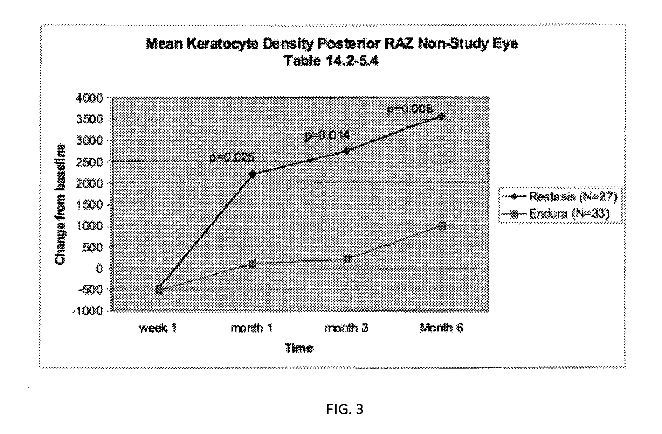

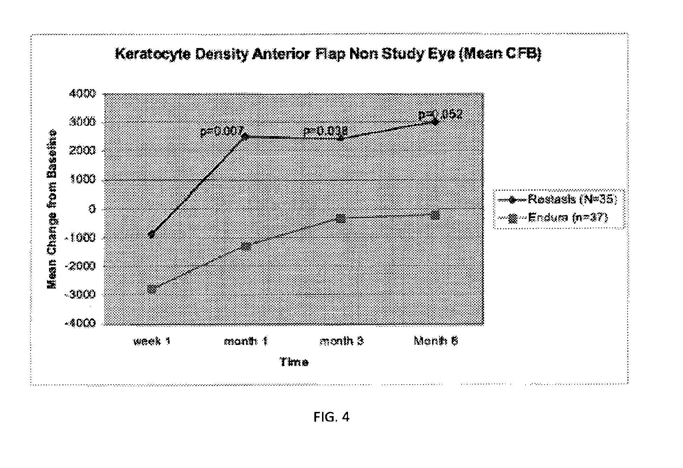

Keratocyte density and Goblet cell density were as shown in Figures 1-4.

The invention may be summarized as follows:
1. A method of treating a patient having blurred vision, the method comprising administering a cyclosporin composition to an eye of a subject having blurred vision.
2. A method of increasing keratocyte density, the method comprising administering a cyclosporin composition to an eye of a subject having keratocyte loss or who would otherwise benefit from increased keratocyte density.
3. A method of increasing goblet cell density, the method comprising administering a cyclosporin composition to an eye of a subject having goblet cell loss or who would otherwise benefit from increased goblet cell density.
4. A method of treating psychological distress following surgery on the eye, the method comprising administering a cyclosporin composition to an eye of a subject in need of such treatment.
5. A method of treating a patient having sensitivity to light, the method comprising administering a cyclosporin composition to an eye of a subject in need of such treatment.
6. The method of 1, 2, 3, 4, or 5, wherein the cyclosporin composition comprises cyclosporin A.
7. The method of 6, wherein the cyclosporin composition is topically administered to the surface of the eye at a concentration of from about 0.005% (w/v) to about 2.5% (w/v), from about 0.01% (w/v) to about 1 % (w/v), or from about 0.01% (w/v) to about 0.05% (w/v).
8. The method of 6, wherein the cyclosporin composition is topically administered to the surface of the eye at a concentration of 0.005% (w/v), a concentration of 0.01% (w/v), a concentration of 0.02% (w/v), a concentration of 0.03% (w/v), a concentration of 0.04% (w/v), or a concentration of 0.05% (w/v).
9. The method according to any of the foregoing, wherein 25-50 µg of the cyclosporin composition is administered to the eye.
10. The method of 9, wherein the cyclosporin composition is administered one, two, three, or four times a day.

Embodiments of the invention:
1. A method of treating a patient having blurred vision, the method comprising topically administering a cyclosporin composition to an eye of a subject having blurred vision.
2. The method of 1, wherein the cyclosporin composition comprises cyclosporin A at a concentration from about 0.01% (w/v) to about 0.05% (w/v).
3. A method of increasing keratocyte density, the method comprising administering a cyclosporin composition to an eye of a subject having keratocyte loss or who would otherwise benefit from increased keratocyte density.
4. The method of 3, wherein the cyclosporin composition comprises cyclosporin A at a concentration from about 0.01% (w/v) to about 0.05% (w/v).
5. A method of increasing goblet cell density, the method comprising administering a cyclosporin composition to an eye of a subject having goblet cell loss or who would otherwise benefit from increased goblet cell density.
6. The method of 5, wherein the cyclosporin composition comprises cyclosporin A at a concentration from about 0.01% (w/v) to about 0.05% (w/v).
7. A method of treating psychological distress following surgery on the eye, the method comprising administering a cyclosporin composition to an eye of a subject in need of such treatment.
8. The method of 7, wherein the cyclosporin composition comprises cyclosporin A at a concentration from about 0.01 % (w/v) to about 0.05% (w/v).
9. A method of treating a patient having sensitivity to light, the method comprising topically administering a cyclosporin composition to an eye of a patient in need of such treatment.
10. The method of 9, wherein the cyclosporin composition comprises cyclosporin A at a concentration from about 0.01% (w/v) to about 0.05% (w/v).

## Claims

1. A cyclosporin composition for use in a method of treating blurred vision by topical administration to the eye.

2. A cyclosporin composition for use according to Claim 1, wherein the composition comprises cyclosporin A at a concentration of 0.01-0.05% (w/v).

3. A cyclosporin composition for use according to Claim 1 or Claim 2, wherein the blurred vision is caused by a disease of the eye.

4. A cyclosporin composition for use according to Claim 1 or Claim 2, wherein the blurred vision is iatrogenic.

5. A cyclosporin composition for use according to Claim 4, wherein the blurred vision is caused by a surgical procedure.
